# EUROPÄISCHE PATENTSCHRIFT

(11) **EP 1 173 095 B1**
(45) Veröffentlichungstag und Bekanntmachung des Hinweises auf die Patenterteilung: **23.06.2004**
(21) Anmeldenummer: 00918567.9
(22) Anmeldetag: 19.04.2000
(51) Int. Cl.: A61B 5/0408, A61N 1/32, A61B 18/16

(54) **MEDIZINISCHE ELEKTRODE**
MEDICAL ELECTRODE
ELECTRODE MEDICALE

(30) Priorität: 29.04.1999 AT 76999
(43) Veröffentlichungstag der Anmeldung: 23.01.2002
(73) Patentinhaber: Leonhard Lang KG, 6010 Innsbruck (AT)
(72) Erfinder: LANG, Burrhus, A-6020 Innsbruck (AT); LANG, Sergius, A-6020 Innsbruck (AT)
(74) Vertreter: Torggler, Paul Norbert, Dr.
(86) Internationale Anmeldenummer: PCT/AT2000/000098
(87) Internationale Veröffentlichungsnummer: WO 2000/065993

(56) Entgegenhaltungen:
- CA-A- 1 219 642
- DE-A- 4 231 236
- US-A- 5 114 424

## Beschreibung

Die Erfindung betrifft eine medizinische Elektrode, insbesondere zur Energieübertragung mit mindestens einer elektrisch kontaktierbaren, vorzugsweise mit einer Anschlußlasche oder dgl. versehenen Leiterfläche.

Solche Elektroden werden zu verschiedensten Zwecken auf die Haut des Patienten aufgeklebt, beispielsweise um bioelektrische Vorgänge des Körpers zu überwachen oder um - meist höher frequente Ströme - aus dem Körper einzuleiten bzw. abzuleiten (zB Neutralelektroden, Stimulationselektroden und Defibrillationselektroden). Der Aufbau dieser Elektroden kann verschiedenartig sein, im allgemeinen weisen solche Elektroden einen von der Haut entfernten rückwärtigen Träger aus einem Schaumstoffmaterial auf. An diesem sind gegebenenfalls unter Zwischenschaltung von Zwischenschichten elektrisch leitfähige Leiterflächen vorgesehen, beispielsweise ein Aluminiumlaminat. Es können jedoch auch nichtmetallische Leiterflächen vorgesehen sein. Im Falle von Neutralelektroden stehen diese Leiterflächen zur Vermeidung des Auftretens von hohen lokalen Stromdichten nicht direkt mit der Haut in Kontakt. Vielmehr ist ein für die angewandten Wechselströme elektrisch leitendes, klebriges Gel vorgesehen, das den Kontakt zur Haut herstellt.

Bei Neutralelektroden zur Stromableitung von einem Operationsfeld ist es bereits bekannt, diese Elektroden mit mindestens zwei elektrisch getrennten Leiterflächen auszustatten, wobei eine elektronische Auswerteinrichtung die von den jeweiligen Leiterflächen abgeleiteten Ströme einzeln überwacht und bei Feststellen einer zu großen Differenz einen Alarm gibt. Zweck dieser Vorgangsweise ist es, sicherzustellen, daß beide Leiterflächen der Neutralelektrode einen guten elektrischen Kontakt zur Haut haben, um lokale Wärmeentwicklungen an der Haut des Patienten auszuschließen. Bei der bekannten Neutralelektrode sind beispielsweise zwei im wesentlichen rechteckige Leiterflächen vorgesehen, die mit einem dazwischenliegenden Spalt nebeneinander auf einem gemeinsamen Träger angeordnet sind. Damit diese Neutralelektrode samt der angeschlossenen Überwachungseinrichtung funktioniert, muß der Spalt exakt zum Operationsfeld ausgerichtet sein, da sonst die beiden Leiterflächen unterschiedlich mit Strom beaufschlagt werden. Eine solche Elektrode ist in CA-1 219 642 offenbart.

Um die Stromaufteilung, insbesondere bei stromableitenden Neutralelektroden zu verbessern und gleichmäßiger zu gestalten, ist erfindungsgemäß vorgesehen, daß wenigstens eine unkontaktierte Leiterfläche vorgesehen ist, die mit Abstand und elektrisch getrennt von der mindestens einen elektrisch kontaktierbaren Leiterfläche angeordnet ist.

Die frei von Anschlußlaschen ausgebildete unkontaktierte Leiterfläche kann beispielsweise die kontaktierte Leiterfläche kreisringförmig umgeben. Es können auch zwei oder mehrere solcher unkontaktierten Leiterflächen auf einem gemeinsamen Träger mit der bzw. den kontaktierbaren Leiterflächen vorgesehen sein. Auch ist es möglich, daß die unkontaktierte Leiterfläche in den Zwischenraum zwischen zwei beabstandeten kontaktierten Leiterflächen reicht.

Aufgabe dieser unkontaktierten Leiterflächen ist es - wie bereits erwähnt - die Stromaufteilung, insbesondere bei stromableitenden Neutralelektroden, zu verbessern und gleichmäßiger zu gestalten. Vor allem bei solchen Neutralelektroden, die bevorzugt zwei oder mehrere elektrisch kontaktierbare Leiterflächen aufweisen, kann ein zusätzlicher, nichtkontaktierter Leiterflächenring zur gleichmäßigen Aufteilung des abzuleitenden Stromes auf die beiden Teilelektroden (Leiterflächen) führen. Es ergibt sich somit insgesamt eine bessere Stromdichteverteilung und damit eine geringere Wärmebelastung für den Patienten.

Um eine medizinische Elektrode mit mindestens zwei elektrisch getrennten Leiterflächen zu schaffen, die eine gleichmäßige Erfassung von Biopotentialen oder Energieübertragung erlauben, ist gemäß einem bevorzugten Ausführungsbeispiel vorgesehen, daß eine Leiterfläche eine andere Leiterfläche - in einer Draufsicht gesehen - zumindest teilweise umgibt.

Die innere Leiterfläche ist vorzugsweise kreisrund und die äußere Leiterfläche umgibt diese innere Leiterfläche kreisringförmig. Der Spalt zwischen den beiden elektrisch getrennten Leiterflächen verläuft dann als Ringspalt zwischen der inneren und der äußeren Leiterfläche. Durch entsprechende Dimensionierung und Ausbildung ist es gemäß einer Ausführungsform möglich, die Flächeninhalte und/oder Umfangslängen der beiden in der Gestalt doch unterschiedlichen Leiterflächen dennoch im wesentlichen gleich auszubilden, insbesondere um im Fall der Neutralelektrode im wesentlichen gleiche Ableitungsbedingungen zu schaffen und eine hohe Ausrichtungstoleranz zu gewährleisten.

Ein wesentliche Vorteil einer solchen bevorzugten Elektrodenausbildung besteht darin, daß diese, abgesehen von einer kompakten Bauform, in vielen unterschiedlichen Orientierungen auf die Haut geklebt werden kann, ohne eine wesentliche Veränderung in der Stromleitfähigkeit in Kauf nehmen zu müssen (hohe Ausrichtungstoleranz, das heißt eine flexible Orientierbarkeit beispielsweise relativ zu einem Operationsfeld). Dabei ist es besonders günstig, wenn die äußere Leiterfläche die innere auf einem Winkelbereich von über 90°, vorzugsweise über 270°, umgibt. Während man bei der bisherigen Neutralelektrode nach dem Stand der Technik den Spalt immer genau zum Operationsfeld ausrichten mußte, kann nunmehr das medizinische Personal die neuartige Elektrode in nahezu beliebiger Orientierung auf die Haut kleben. Dies erleichtert die Anwendung erheblich.

Trotz der Tatsache, daß die Leiterflächen mit ihren aktiven Bereichen sich einander umgeben, ist es günstig, die Anschlußlappen seitlich nebeneinander parallel heraus zuführen, um einen einfachen Anschluß des mehrpoligen Elektrodenkabels zu erlauben.

Eine weitere Ausführungsform der Erfindung geht von der Erkenntnis aus, daß an Ecken der leitfähigen Bereiche höhere lokale Stromdichten auftreten können. Um dies zu vermeiden, sieht diese Ausführungsform der Erfindung vor, daß die leitfähigen Bereiche im wesentlichen rund, vorzugsweise kreisrund ausgebildet sind. Damit können die ungünstigen Ecken vermieden werden und außerdem eine Unempfindlichkeit gegen unterschiedliche Orientierungen beim Aufbringen der Elektrode sichergestellt werden.

Weitere Vorteile und Einzelheiten der Erfindung werden anhand der nachfolgenden Figurenbeschreibung näher erläutert.

Die Fig. 1 zeigt schematisch die Anordnung von zwei elektrisch getrennten Leiterflächen in einer Elektrode, wobei der Träger, beispielsweise eine klebrige Schaumstoffaulage, strichliert dargestellt ist.

Die Figuren 2 bis 11 zeigen weitere Anordnungen von Leiterflächen für eine Elektrode, insbesondere Neutralelektrode, wobei Trägermaterialien bzw. mögliche hautseitige, elektrisch leitende, klebrige Gels der Einfachkeit halber nicht dargestellt sind. Die Figuren 4, 6, 7, 8, 9, 10 und 11 zeigen dabei die erfindungsgemäß unkontaktierte Leiterfläche.

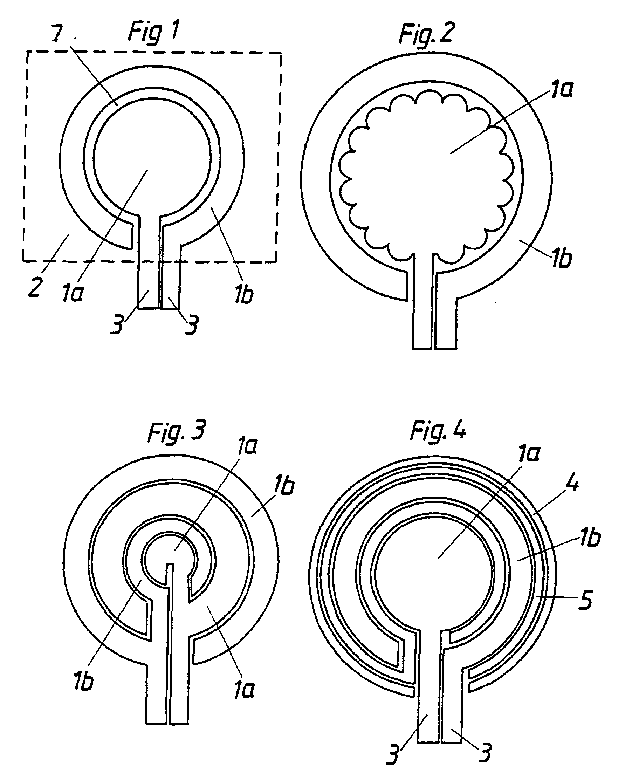

Die in Fig. 1 gezeigte medizinische Hautelektrode weist auf einem Träger 2 zwei elektrisch getrennte Leiterflächen 1a und 1b auf, die mit Anschlußlaschen 3 versehen sind. Die äußere Leiterfläche 1b umgibt die innere Leiterfläche 1a, wie dies in einer Draufsicht gemäß Fig. 1 zu sehen ist. Die innere Leiterfläche 1a ist im wesentlichen kreisrund und die äußere Leiterfläche 1b im wesentlichen kreisringförmig, wobei dazwischen ein Spalt 7 konstanter Breite angeordnet ist. Es ist besonders günstig, wenn die äußere Leiterfläche 1b die innere auf einem möglichst großen Winkelbereich umgibt. Dieser sollte mindestens 90°, vorzugsweise über 270° betragen. Mit einer solchen Ausbildung ist es möglich, die Elektrode in nahezu beliebiger Ausrichtung gegenüber dem Operationsfeld anzuordnen und dennoch immer eine sichere und gleichmäßig auf die beiden Teilflächen 1a und 1b verteilte Stromableitung zu erzielen. Bei Anschluß eines zum Stand der Technik gehörenden Überwachungsgerätes, das die relativen Ströme aus den beiden Leiterflächen 1a und 1b mißt, kommt es somit beim Aufkleben der Elektrode in nahezu beliebiger Orientierung relativ zum Operationsfeld nicht zu einem Auslösen eines ungewünschten Alarms. Die Elektrode kann damit vom medizinischen Fachpersonal rasch und unkompliziert angebracht werden.

Um für die Stromableitung (allgemein: Energieübertragung) für die beiden Leiterflächen 1a und 1b möglichst identische Bedingungen zu schaffen, sind die Flächeninhalte der beiden Flächen 1a und 1b hier gleich gewählt.

Bei der in Fig. 2 dargestellten Elektrode weist die innere Leiterfläche 1b eine mehrfach bombierte Außenkante auf, um deren Umfangslänge so zu erhöhen, daß sie im wesentlichen der Umfangslänge des äußeren hakenförmigen bzw. kreisringförmigen Leiterflächenelementes 1b entspricht.

Die Fig. 3 zeigt eine "Doppelhaken-Geometrie", bei der die Leiterflächen 1a und 1b hakenförmige Vorsprünge aufweisen, die ineinander verschachtelt sind, um eine gleichmäßige Stromverteilung auf die beiden Halbelektroden zu erzielen.

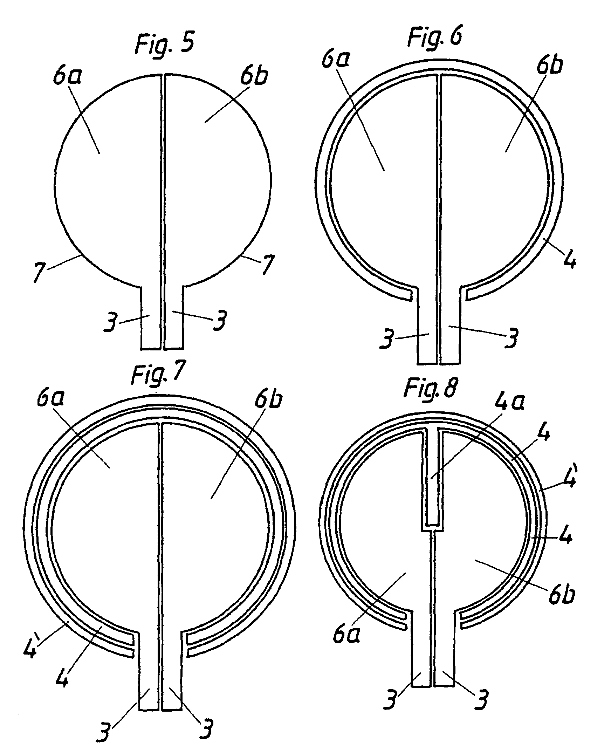

Bei der in Fig. 4 dargestellten Elektrode sind ebenfalls zwei elektrisch kontaktierte Leiterflächen 1a und 1b vorgesehen, die ineinander verschachtelt sind bzw. sich zumindest teilweise umgeben. Gemäß der Erfindung sind bei dieser Elektrode noch zwei unkontaktierte Ringe 4 und 5 vorgesehen, die im Gegensatz zu den Leiterflächen 1a und 1b keine Anschlußelemente 3 für ein Elektrodenkabel aufweisen. Der äußere unkontaktierte Ring umschließt alle inneren Leiterflächen, während der innere unkontaktierte Ring zusätzlich noch in den Spalt zwischen den beiden kontaktierten Leiterflächen 1a und 1b (den eigentlich aktiven Elektrodenflächen) reicht. Zweck derartiger unkontaktierter Leiterflächen bzw. Ringe 4, 5 ist es, eine gleichmäßige Stromaufteilung zu erzielen. Versuche am Patienten mit Neutralelektroden haben gezeigt, daß es durch den Einsatz solcher unkontaktierter Ringe zu einer wesentlich geringeren Wärmebelastung durch eine verbesserte Stromdichteverteilung kommt.

Günstigerweise wird man diese unkontaktierten Ringe und die kontaktierten Leiterflächen 1a und 1b auf einem in Fig. 4 nicht dargestellten Träger, beispielsweise aus Schaumstoff, anordnen und falls dies gewünscht ist, mit einem hautseitig elektrisch leitenden Gel abdecken. Grundsätzlich ist es jedoch auch möglich, die unkontaktierten, elektrisch leitenden Ringe bzw. die kontaktierbaren Leiterflächen 1a und 1b unabhängig von einander am Patienten als gesonderte Bauelemente anzubringen.

Um vorhandene Ecken an rechteckigen Elektrodenteilen zu vermeiden, wählt man die Form der Leiterflächen günstigerweise so, daß sie eine runde, vorzugsweise eine kreisrunde Außenkontur aufweisen (mit Ausnahme der Anschlußlaschen 3). Eine solche Ausführungsform ist in der Fig. 5 schematisch gezeigt, wo die beiden Leiterflächen 6a und 6b eine klar ersichtliche kreisrunde Außenkontur 7 aufweisen. Eine solche einfache, runde, zweiflächige Doppelelektrode kann natürlich auch mit einem zusätzlichen unkontaktierten Ring 4 umgeben sein, der die Außenkontur zumindest teilweise umschließt. Damit kann wiederum die Erwärmung der Elektrode beim Stromfluß im Zuge des medizinischen Einsatzes besonders gering und gleichmäßig gehalten werden. Bei dem in Fig. 7 dargestellten Ausführungsbeispiel befindet sich außerhalb des unkontaktierten Ringes 4 noch ein weiterer Ring 4', also insgesamt zwei unkontaktierte Ringe, die zu einer weiteren Vergleichmäßigung des Stromflusses bei der Anwendung führen. Es ist auch möglich, daß die unkontaktierte Leiterfläche 4 einen Fortsatz 4a aufweist, der in den Bereich zwischen den beiden elektrisch kontaktierten Leiterflächen reicht.

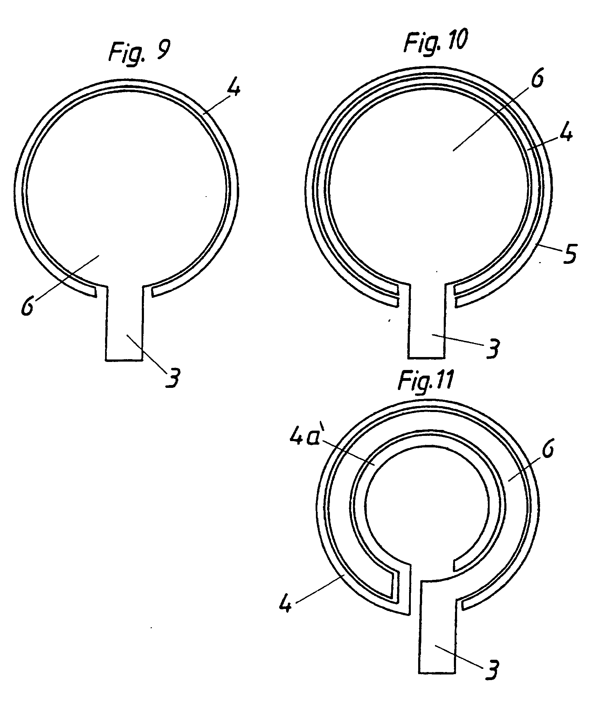

Die Idee einer medizinischen Elektrode mit einer elektrisch unkontaktierten, vorzugsweise ringförmigen Leiterfläche 4 bzw. 5 läßt sich auch bei Elektroden mit nur einer elektrisch kontaktierten Leiterfläche 6 verwirklichen, wie dies die Figuren 9, 10 und 11 zeigen. Zur Fig. 11 ist noch zu erwähnen, daß hier die stromführende kontaktierte Elektrode 6 im wesentlichen hakenförmig ausgebildet ist, wobei sich der kontaktlose Außenring 4 mit einem Fortsatz 4'a nach innen erstreckt und damit auch die Innenseite der Hakenelektrode abdeckt.

## Patentansprüche

1. Medizinische Elektrode mit mindestens einer elektrisch kontaktierbaren, vorzugsweise mit einer Anschlußlasche versehenen Leiterfläche, **dadurch gekennzeichnet, daß** wenigstens eine unkontaktierte Leiterfläche (4, 5) vorgesehen ist, die mit Abstand und elektrisch getrennt von der mindestens einen elektrisch kontaktierbaren Leiterfläche (1a, 1b) angeordnet und frei von Anschlußlaschen (3) ist.

2. Medizinische Elektrode nach Anspruch 1, **dadurch gekennzeichnet, daß** die mindestens eine elektrisch kontaktierbare Leiterfläche (1a, 1b) und mindestens eine unkontaktierte Leiterfläche (4, 5) auf einem gemeinsamen Träger (2) angeordnet sind.

3. Medizinische Elektrode nach einem der Ansprüche 1 oder 2, **dadurch gekennzeichnet, daß** eine der unkontaktierten Leiterflächen (4, 5) eine oder mehrere der anderen kontaktierten Leiterflächen (1a, 1b), vorzugsweise kreisringförmig, zumindest teilweise umgibt bzw. entlang dieser verläuft.

4. Medizinische Elektrode nach einem der Ansprüche 1 bis 3, **dadurch gekennzeichnet, daß** die unkontaktierte Leiterfläche in den Zwischenraum zwischen zwei beabstandete kontaktierte Leiterflächen (1a, 1b) oder in eine Einbuchtung in einer Leiterfläche reicht.

5. Medizinische Elektrode nach einem der Ansprüche 1 bis 4, **dadurch gekennzeichnet, daß** zwei, zumindest abschnittweise parallel gekrümmte, unkontaktierte Leiterabschnitte (4, 5) vorgesehen sind.

6. Medizinische Elektrode nach einem der Ansprüche 1 bis 5, **dadurch gekennzeichnet, daß** mindestens zwei elektrisch getrennte, kontaktierbare Leiterflächen vorgesehen sind, wobei eine dieser Leiterflächen (1b) eine andere dieser Leiterflächen (1a), in einer Draufsicht gesehen, zumindest teilweise umgibt.

7. Medizinische Elektrode nach Anspruch 6, **dadurch gekennzeichnet, daß** bei diesen getrennten Leiterflächen eine innere Leiterfläche (1a) von einer äußeren Leiterfläche (1b) umgeben ist, welche vorzugsweise mit konstantem Spaltabstand zum Außenrand der inneren Leiterfläche (1a) um diese herumläuft.

8. Medizinische Elektrode nach Anspruch 6 oder 7, **dadurch gekennzeichnet, daß** bei diesen elektrisch getrennten kontaktierbaren Leiterflächen eine innere Leiterfläche (1a) im wesentlichen kreisrund ausgebildet und von einer kreisförmigen äußeren Leiterfläche umgeben ist.

9. Medizinische Elektrode nach einem der Ansprüche 6 bis 8, **dadurch gekennzeichnet, daß** bei diesen elektrisch getrennten kontaktierbaren Leiterflächen die äußere Leiterfläche (1b) die innere (1a) auf einem Winkelbereich von über 90°, vorzugsweise von über 270°, umgibt.

10. Medizinische Elektrode nach einem der Ansprüche 6 bis 9, **dadurch gekennzeichnet, daß** bei diesen elektrisch getrennten kontaktierbaren Leiterflächen zumindest eine innere (1a) und eine diese umgebende äußere (1b) Leiterfläche jeweils eine abstehende Anschlußlasche (3) für ein Elektrodenkabel aufweisen, wobei die Anschlußlaschen (3) vorzugsweise seitlich nebeneinander und parallel zueinander angeordnet sind.

11. Medizinische Elektrode nach einem der Ansprüche 6 bis 10, **dadurch gekennzeichnet, daß** in unterschiedlicher radialer Lage zwei elektrisch kontaktierbare Leiterflächen (1a, 1b) vorgesehen sind, deren Flächeninhalte und/oder Umfangslängen im wesentlichen gleich sind.

12. Medizinische Elektrode nach einem der Ansprüche 6 bis 11, **dadurch gekennzeichnet, daß** bei diesen elektrisch getrennten kontaktierbaren Leiterflächen zumindest eine Leiterfläche (1b) hakenförmig ausgebildet ist, wobei der Haken (1b) die andere Leiterfläche (1a) umgibt.

13. Medizinische Elektrode nach einem der Ansprüche 6 bis 12, **dadurch gekennzeichnet, daß** bei diesen elektrisch getrennten kontaktierbaren Leiterflächen jede Leiterfläche (1a, 1b) vorzugsweise hakenförmige Vorsprünge aufweist, die ineinander verschachtelt sind. (Fig. 3)

14. Medizinische Elektrode nach einem der Ansprüche 1 bis 13, **dadurch gekennzeichnet, daß** die Außenkontur der Leiterfläche(n) (6, 6a, 6b) rund ist.

## Claims

1. A medical electrode comprising at least one electrically contactable conductor surface preferably provided with a connecting bar, **characterised in that** there is at least one uncontacted conductor surface (4, 5) which is arranged at a spacing and electrically separated from the at least one electrically contactable conductor surface (1a, 1b) and which is free from connecting bars (3).

2. A medical electrode as set forth in claim 1, **characterised in that** the at least one electrically contactable conductor surface (1a, 1b) and at least one uncontacted conductor surface (4, 5) are arranged on a common carrier (2).

3. A medical electrode as set forth in one of claim 1 or 2, **characterised in that** one of the uncontacted conductor surfaces (4, 5) at least partially surrounds one or more of the other contacted conductor surfaces (1a, 1b), preferably in the form of a circular ring, or extends along same.

4. A medical electrode as set forth in one of claims 1 through 3, **characterised in that** the uncontacted conductor surface extends into the intermediate space between two spaced contacted conductor surfaces (1a, 1b) or into a recess configuration in a conductor surface.

5. A medical electrode as set forth in one of claims 1 through 4, **characterised in that** there are provided two uncontacted conductor portions (4, 5) which are curved parallel at least in a portion-wise manner.

6. A medical electrode as set forth in one of claims 1 through 5, **characterised in that** there are provided at least two electrically separated contactable conductor surfaces, wherein one of said conductor surfaces (1b) at least partially surrounds another of said conductor surfaces (1a), as viewed in plan.

7. A medical electrode as set forth in claim 6, **characterised in that** concerning these separated conductor surfaces, an inner conductor surface (1a) is surrounded by an outer conductor surface (1b) which preferably extends around the inner conductor surface (1a) at a constant gap spacing relative to the outer edge thereof.

8. A medical electrode as set forth in claim 6 or claim 7, **characterised in that**, concerning these electrically separated conductor surfaces, an inner conductor surface (1a) is of a substantially round circular configuration and is surrounded by an outer conductor surface in the form of a circular ring.

9. A medical electrode as set forth in one of claims 6 through 8, **characterised in that**, concerning these electrically separated conductor surfaces, the outer conductor surface (1b) surrounds the inner (1a) over an angular range of over 90°, preferably over 270°.

10. A medical electrode as set forth in one of claims 6 through 9, **characterised in that**, concerning these electrically separated conductor surfaces, at least one inner conductor surface (1a) and an outer conductor surface (1b) surrounding same each have a respective projecting connecting bar (3) or the like for an electrode cable, wherein the connecting bars (3) are preferably arranged laterally one beside the other and parallel to each other.

11. A medical electrode as set forth in one of claims 6 through 10, **characterised in that** there are provided two electrically contactable conductor surfaces (1a, 1b) in different radial positions, the surface areas and/or peripheral lengths thereof being substantially equal.

12. A medical electrode as set forth in one of claims 6 through 11, **characterised in that**, concerning these electrically separated conductor surfaces, at least one conductor surface (1b) is of a hook-shaped configuration, the hook (1b) surrounding the other conductor surface (1a).

13. A medical electrode as set forth in one of claims 6 through 12, **characterised in that**, concerning these electrically separated conductor surfaces, each conductor surface (1a, 1b) has preferably hook-shaped projections which are interleaved one into the other (Figure 3).

14. A medical electrode as set forth in one of claims 1 through 13, **characterised in that** the outside contour of the conductor surface or surfaces (6, 6a, 6b) is round.

## Revendications

1. Electrode médicale présentant au moins une surface conductrice pouvant être connectée électriquement à un câble d'électrodes et pourvue de préférence d'une languette de raccordement, **caractérisée en ce qu'**au moins une surface conductrice non connectée (4, 5) est prévue, qui est située à distance et isolée électriquement de la au moins une surface conductrice (1a, 1b) pouvant être connectée électriquement et qui est exempte de languette de raccordement (3).

2. Electrode médicale selon la revendication 1, **caractérisée en ce que** la au moins une surface conductrice pouvant être connectée électriquement (1a, 1b) et la surface conductrice non connectée (4, 5) sont disposées sur un support commun (2).

3. Electrode médicale selon l'une des revendications 1 ou 2, **caractérisée en ce que** l'une des surfaces non connectées (4, 5) entoure au moins en partie et de préférence en forme de couronne torique une ou plusieurs des autres surfaces conductrices connectées (1a, 1b) ou s'étend le long de celles-ci.

4. Electrode médicale selon l'une des revendications 1 à 3, **caractérisée en ce que** la surface conductrice non connectée s'étend dans l'espacement entre deux surfaces conductrices connectées isolées (1a, 1b) ou dans une rainure dans une surface conductrice.

5. Electrode médicale selon l'une des revendications 1 à 4, **caractérisée en ce que** deux sections conductrices (4, 5) non connectées, coudées parallèlement au moins en partie sont prévues.

6. Electrode médicale selon l'une des revendications 1 à 5, **caractérisée en ce qu'**au moins deux surfaces conductrices isolées, pouvant être connectées électriquement sont prévues, et où l'une de ces surfaces conductrices (1b) entoure au moins partiellement, vu de dessus, une autre de ces surfaces conductrices (1a).

7. Electrode médicale selon la revendication 6, **caractérisée en ce que**, pour ces surfaces conductrices isolées, une surface conductrice interne (1a) est entourée d'une surface conductrice externe (1b), laquelle s'étend autour de la surface conductrice interne (1a) de préférence avec un écartement constant par rapport au bord externe de celle-ci.

8. Electrode médicale selon la revendication 6 ou 7, **caractérisée en ce que**, pour ces surfaces conductrices isolées, pouvant être connectées électriquement, une surface conductrice interne (1a) est essentiellement circulaire et est entourée d'une surface conductrice externe circulaire.

9. Electrode médicale selon l'une des revendications 6 à 8, **caractérisée en ce que**, pour ces surfaces conductrices isolée, pouvant être connectées électriquement, la surface conductrice externe (1b) entoure la surface interne (1a) sur une zone angulaire supérieure à 90°, de préférence supérieure à 270°.

10. Electrode médicale selon l'une des revendications 6 à 9, **caractérisée en ce que**, pour ces surfaces conductrices isolées, pouvant être connectées électriquement, au moins une surface conductrice interne (1a) et une surface conductrice externe (1b) entourant celle-ci présentent respectivement une languette de raccordement de prolongation (3), pour un câble d'électrodes, les languettes de raccordement (3) sont disposés de préférence latéralement l'une à côté de l'autre et parallèlement l'une par rapport à l'autre.

11. Electrode médicale selon l'une des revendications 6 à 10, **caractérisée en ce que** deux surfaces conductrices (1a, 1b) pouvant être connectées électriquement, dont les superficies et/ou les circonférences sont essentiellement égales, sont prévues en position radiale différente.

12. Electrode médicale selon l'une des revendications 6 à 11, **caractérisée en ce que**, pour ces surfaces conductrices isolées, pouvant être connectées électriquement, au moins une surface conductrice (1b) est en forme de crochet, et où le crochet, et où le crochet (1b) entoure l'autre surface conductrice (1a).

13. Electrode médicale selon l'une des revendications 6 à 12, **caractérisée en ce que**, pour ces surfaces conductrices isolées, pouvant être connectées électriquement, chaque surface conductrice (1a, 1b) présente des saillies de préférence en forme de crochet, qui sont imbriquées les unes dans les autres (figure 3).

14. Electrode médicale selon l'une des revendications 1 à 13, **caractérisée en ce que** le contour extérieur de la (des) surface(s) conductrice(s) (6, 6a, 6b) est circulaire.
